# EUROPEAN PATENT SPECIFICATION

(11) **EP 4 020 054 B1**
(45) Date of publication and mention of the grant of the patent: **21.02.2024**
(21) Application number: 20855251.3
(22) Date of filing: 16.04.2020
(51) Int. Cl.: G02B 23/24, A61B 1/00

(54) **ENDOSCOPE SYSTEM AND OPERATION METHOD THEREFOR**
ENDOSKOPSYSTEM UND BETRIEBSVERFAHREN DAFÜR
SYSTÈME D'ENDOSCOPE ET SON PROCÉDÉ DE FONCTIONNEMENT

(30) Priority: 20.08.2019 JP 2019150387
(43) Date of publication of application: 29.06.2022
(73) Proprietor: FUJIFILM Corporation, Tokyo 106-8620 (JP)
(72) Inventor: KAGAYA, Makoto, Ashigarakami-gun, Kanagawa 258-8538 (JP)
(74) Representative: Hughes, Andrea Michelle
(86) International application number: PCT/JP2020/016695
(87) International publication number: WO 2021/033367

(56) References cited:
- WO-A1-2017/217162
- JP-A- 2003 245 243
- JP-A- 2018 171 356
- US-A1- 2012 182 127
- US-A1- 2019 082 930

## Description

### BACKGROUND OF THE INVENTION

### 1. Field of the Invention

The present disclosure relates to an endoscope system and an operation method thereof.

### 2. Description of the Related Art

A detection device that measures a magnetic field generated by a plurality of magnetic field generation elements by a magnetic field detection element and detects a shape of an insertion part of an endoscope based on a measurement result is disclosed (see JP2018-171356A). In this detection device, driving order of the magnetic field generation elements within a predetermined measurement unit time is always the same order.

US 2019/082 930 A1 discloses an endoscope system that generates magnetic fields by selectively switching and driving a plurality of magnetic field generation elements.

### SUMMARY OF THE INVENTION

The invention is defined by the independent claims, with preferred embodiments set out in the dependent claims.

In a case in which the driving order of the magnetic field generation elements is always the same order within the measurement unit time, a driving period of each magnetic field generation element is also fixed. Therefore, a periodic noise may have a large influence only on the magnetic field generated by the same specific magnetic field generation element. In this case, the magnetic field generated by the magnetic field generation element includes a relatively large influence of the noise, and as a result, an insertion state including at least one of a position or a shape of an insertion part of an endoscope cannot be detected with high accuracy.

The present disclosure has been made in view of the above circumstances, and is to provide an endoscope system and an operation method thereof which can detect an insertion state including at least one of a position or a shape of an insertion part of an endoscope with high accuracy.

An aspect of the present disclosure relates to an endoscope system that generates magnetic fields by selectively switching and driving a plurality of magnetic field generation elements, and detects an insertion state including at least one of a position or a shape of an insertion part of an endoscope based on measurement results of the magnetic fields by magnetic field detection elements, the system comprising a magnetic field generation control unit that performs a control of varying driving order of the magnetic field generation elements within a predetermined measurement unit time between a plurality of the measurement unit times.

Note that the endoscope system according to the present disclosure may further comprise a magnetic field measurement control unit that performs a control of varying order of acquiring, from a plurality of the magnetic field detection elements, the measurement results of the magnetic fields generated within a unit driving time in which one magnetic field generation element is driven within the measurement unit time between a plurality of the unit driving times in a case in which the measurement results of the magnetic fields detected by the plurality of magnetic field detection elements are selectively switched and acquired.

In addition, the endoscope system according to the present disclosure may further comprise an insertion state detection unit that detects the insertion state based on a measurement result obtained by averaging the measurement results of the magnetic fields generated by the plurality of magnetic field generation elements for each of a predetermined number of the latest measurement unit times.

In addition, in the endoscope system according to the present disclosure, the magnetic fields generated by the plurality of magnetic field generation elements may have the same frequency.

In addition, another aspect of the present disclosure relates to an operation method of an endoscope system that generates magnetic fields by selectively switching and driving a plurality of magnetic field generation elements, and detects an insertion state including at least one of a position or a shape of an insertion part of an endoscope based on measurement results of the magnetic fields by magnetic field detection elements, the method comprising a magnetic field generation control step of performing a control of varying driving order of the magnetic field generation elements within a predetermined measurement unit time between a plurality of the measurement unit times.

According to the present disclosure, it is possible to detect the insertion state including at least one of the position or the shape of the insertion part of the endoscope with high accuracy.

### BRIEF DESCRIPTION OF THE DRAWINGS

Fig. 1 is an explanatory diagram showing a state of an endoscopic examination using an endoscope system.
Fig. 2 is a schematic view showing an overall configuration of the endoscope system.
Fig. 3 is a block diagram showing an electric configuration of the endoscope system.
Fig. 4 is a block diagram showing an electric configuration of a magnetic field detection circuit.
Fig. 5 is an explanatory diagram showing a state in which a plurality of detection coils detect a magnetic field generated by a plurality of generation coils.
Fig. 6 is an explanatory diagram showing an example of magnetic field measurement data.
Fig. 7 is a diagram for describing a determination process by a determination unit and a position detection process of each detection coil by a position detection unit.
Fig. 8 is an explanatory diagram showing an example of a shape detection process of an insertion part.
Fig. 9 is a diagram for describing driving of the generation coils in the same order.
Fig. 10 is a diagram for describing driving of the generation coils in the same order.
Fig. 11 is a diagram for describing an influence of a periodic noise.
Fig. 12 is a diagram for describing driving order of the generation coils according to an embodiment.
Fig. 13 is a diagram showing an example of a correspondence relationship for each magnetic field measurement period according to the embodiment.
Fig. 14 is a flowchart showing an example of a display process of an observation image and an insertion part shape image.
Fig. 15 is a diagram for describing a reduction of the influence of the periodic noise.
Fig. 16 is a diagram for describing selection order of magnetic field detection elements.

### DESCRIPTION OF THE PREFERRED EMBODIMENTS

Hereinafter, an embodiment for carrying out the technology of the present disclosure will be described in detail with reference to the drawings.

### <Overall Configuration of Endoscope System>

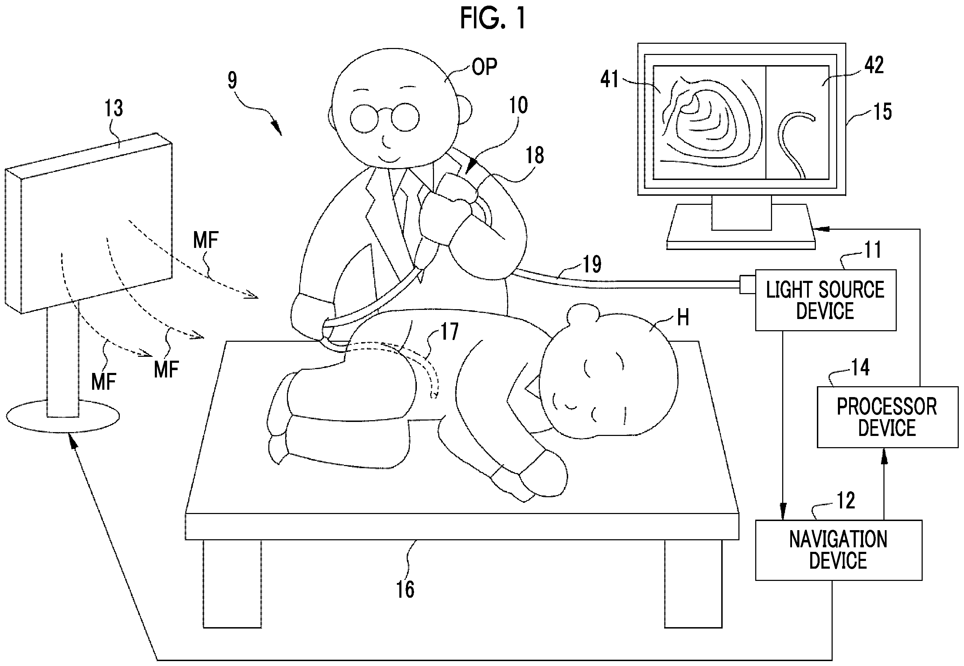

Fig. 1 is a schematic view showing an overall configuration of an endoscope system 9 according to the technology of the present disclosure. As shown in Fig. 1, the endoscope system 9 comprises an endoscope 10, a light source device 11, a navigation device 12, a magnetic field generator 13, a processor device 14, and a monitor 15. The endoscope system 9 is used for an endoscopic examination in a body of a subject H, such as a patient. The subject H is an example of a subject. The endoscope 10 is, for example, an endoscope inserted into the digestive tract, such as the large intestine, and is a soft endoscope having flexibility. The endoscope 10 has an insertion part 17 to be inserted into the digestive tract, an operating part 18 installed consecutively to a proximal end side of the insertion part 17 and gripped by an operator OP to perform various operations, and a universal cord 19 installed consecutively to the operating part 18.

The endoscopic examination is performed, for example, in a state in which the subject H lies on a bed 16. In a case of a large intestine examination, the insertion part 17 of the endoscope 10 is inserted into the digestive tract from an anus by an operator OP who is the doctor. The light source device 11 supplies illumination light that illuminates an inside of the large intestine, which is an observation site, to the endoscope 10. The processor device 14 generates an observation image 41 by processing an image captured by the endoscope 10. The observation image 41 is displayed on the monitor 15. The operator OP proceeds with the endoscopic examination while confirming the observation image 41. The observation image 41 displayed on the monitor 15 is basically a motion picture, but it is also possible to display a still picture as the observation image 41 as needed.

In addition, the endoscope system 9 has a navigation function of navigating technique, such as an insertion operation of the endoscope 10 performed by the operator OP. Here, the navigation means supporting the technique of the endoscope 10 of the operator OP by presenting an insertion state including at least one of the position or the shape of the insertion part 17 of the endoscope 10 in the body of the subject H to the operator OP. The navigation function is a function of presenting the insertion state of the insertion part 17 is detected using a magnetic field MF and the detected insertion state. In the present embodiment, the insertion state includes a position of a part of the insertion part 17 inserted into the body (for example, a distal end part 23 described below), as well as the shape of the entire insertion part 17 in the body.

The navigation function is realized by the navigation device 12, the magnetic field generator 13, and a magnetic field measurement device in the endoscope 10 described below. The magnetic field generator 13 generates the magnetic field MF. The magnetic field generator 13 is attached to, for example, a stand and is disposed beside the bed 16 on which the subject H lies. In addition, the magnetic field generator 13 is disposed within a range in which the generated magnetic field MF reaches the body of the subject H.

The magnetic field measurement device in the endoscope 10 detects the magnetic field MF generated by the magnetic field generator 13 and measures the intensity of the detected magnetic field MF. The navigation device 12 detects the insertion state of the insertion part 17 by deriving a relative position between the magnetic field generator 13 and the insertion part 17 based on a magnetic field measurement result by the magnetic field measurement device. The processor device 14 generates an insertion part shape image 42, which shows the insertion state detected by the navigation device 12.

The monitor 15 displays the observation image 41 and the insertion part shape image 42. Note that the monitor 15 that displays the observation image 41 and the insertion part shape image 42 may be provided separately.

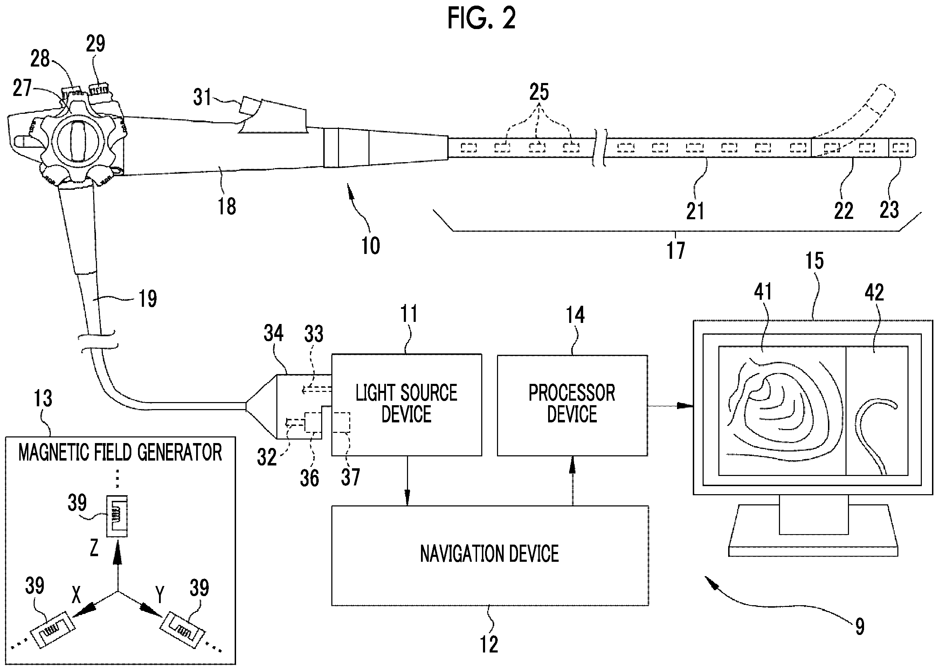

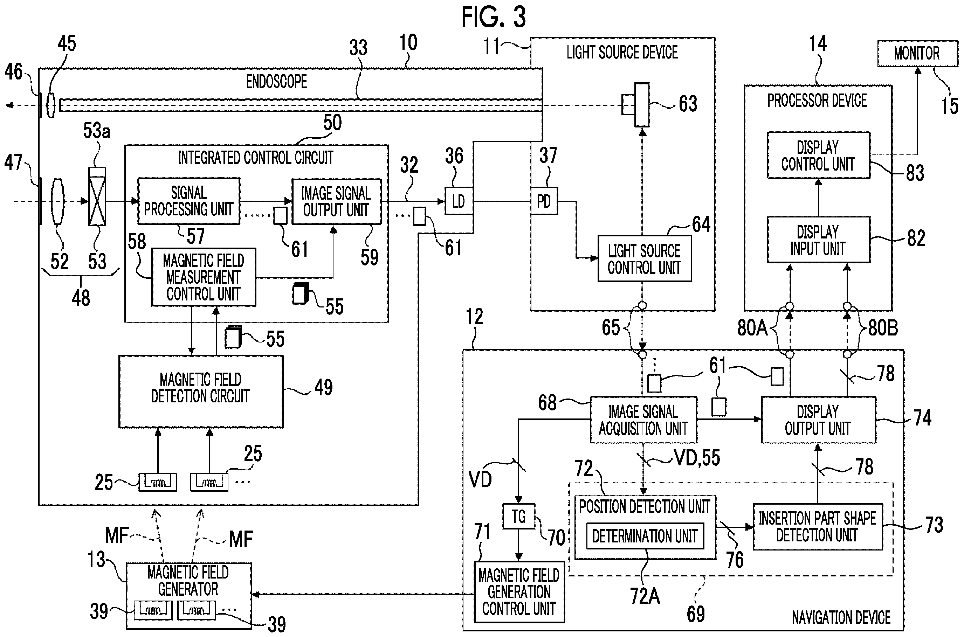

As shown in Fig. 2, the insertion part 17 is a tubular portion having a small diameter and a long length, and is configured by a soft part 21, a bendable part 22, and a distal end part 23, which are connected sequentially from the proximal end side to the distal end side. The soft part 21 has flexibility. The bendable part 22 is a part that can be bent by the operation of the operating part 18. An imaging apparatus 48 (see Fig. 3) and the like are disposed at the distal end part 23. In addition, although not shown, an illumination window 46 (see Fig. 3) that illuminates the observation site with the illumination light, an observation window 47 (see Fig. 3) on which subject light of the illumination light reflected by the subject is incident, a treatment tool outlet for a treatment tool to protrude, and a cleaning nozzle for cleaning the observation window 47 by injecting gas and water into the observation window 47 are provided on a distal end surface of the distal end part 23.

A light guide 33, a signal cable 32, an operation wire (not shown), and a pipe line for inserting the treatment tool (not shown) are provided in the insertion part 17. The light guide 33 extends from the universal cord 19 and guides the illumination light supplied from the light source device 11 to the illumination window 46 at the distal end part 23. The signal cable 32 is used for power supply to the imaging apparatus 48 in addition to communication of an image signal from the imaging apparatus 48 and a control signal for controlling the imaging apparatus 48. Like the light guide 33, the signal cable 32 also extends from the universal cord 19 and is arranged to the distal end part 23.

The operation wire is a wire for operating the bendable part 22, and is arranged from the operating part 18 to the bendable part 22. The pipe line for inserting the treatment tool is a pipe line for inserting the treatment tool (not shown), such as forceps, and is arranged from the operating part 18 to the distal end part 23. In addition, a fluid tube for air/water supply is provided in the insertion part 17. The fluid tube supplies the distal end part 23 with the gas and the water for cleaning the observation window 47.

In addition, in the insertion part 17, a plurality of detection coils 25 are provided from the soft part 21 thereof to the distal end part 23 at preset intervals. Each detection coil 25 corresponds to a magnetic field detection element which detects the magnetic field MF. Each detection coil 25 is affected by the magnetic field MF generated from the magnetic field generator 13, so that an induced electromotive force is generated by an operation of electromagnetic induction, and an induced current is generated by the induced electromotive force. A value of the induced current generated from each detection coil 25 represents the intensity of the magnetic field MF detected by each detection coil 25, which is the magnetic field measurement result. That is, the magnetic field measurement result refers to a value depending on the magnitude of the induced current, which represents the intensity of the magnetic field MF.

The operating part 18 is provided with various operation members operated by the operator OP. Specifically, the operating part 18 is provided with two types of bending operation knobs 27, an air/water supply button 28, and a suction button 29. Each of the two types of bending operation knobs 27 is connected to the operation wire, and is used for a right-left bending operation and an up-down bending operation of the bendable part 22. In addition, the operating part 18 is provided with a treatment tool inlet port 31 which is an inlet of the pipe line for inserting the treatment tool.

The universal cord 19 is a connection cord that connects the endoscope 10 to the light source device 11. The universal cord 19 encompasses the signal cable 32, the light guide 33, and the fluid tube (not shown). In addition, a connector 34 connected to the light source device 11 is provided at an end part of the universal cord 19.

By connecting the connector 34 to the light source device 11, the light source device 11 supplies the power, the control signal, the illumination light, the gas, and the water necessary for operating the endoscope 10 to the endoscope 10. In addition, the image signal of the observation site acquired by the imaging apparatus 48 (see Fig. 3) of the distal end part 23 and the magnetic field measurement result based on a detection signal of each detection coil 25 are transmitted from the endoscope 10 to the light source device 11.

The connector 34 is not electrically connected to the light source device 11 by wire using a metal signal line or the like, and instead, the connector 34 and the light source device 11 are connected to each other so as to be capable of optical communication (non-contact type communication). The connector 34 transmits and receives the control signal exchanged between the endoscope 10 and the light source device 11 and transmits the image signal and the magnetic field measurement result from the endoscope 10 to the light source device 11 by optical communication. The connector 34 is provided with a laser diode (hereinafter, referred to as "LD") 36 connected to the signal cable 32.

The LD 36 is used for transmitting a large amount of data from the endoscope 10 to the light source device 11, specifically, transmitting the image signal and the magnetic field measurement result. The LD 36 transmits the image signal and the magnetic field measurement result, which has been originally in a form of the electric signal, in a form of an optical signal to a photodiode (hereinafter, referred to as "PD") 37 provided in the light source device 11.

Note that, although not shown, apart from the LD 36 and the PD 37, both the connector 34 and the light source device 11 are provided with a light transmission and reception unit that converts a small amount of the control signal exchanged between the endoscope 10 and the light source device 11 into the optical signal and transmits and receives the converted optical signal. Further, the connector 34 is provided with a power reception unit (not shown) that receives the power supplied from a power feed unit (not shown) of the light source device 11 by wireless power supply.

The light guide 33 in the connector 34 is inserted into the light source device 11. In addition, the fluid tube (not shown) in the connector 34 is connected to an air/water supply device (not shown) via the light source device 11. As a result, the light source device 11 and the air/water supply device supply the illumination light, the gas, and the water to the endoscope 10, respectively.

The light source device 11 supplies the illumination light to the light guide 33 of the endoscope 10 via the connector 34, and supplies the gas and the water supplied from the air/water supply device to the fluid tube of the endoscope 10. In addition, the light source device 11 receives the optical signal transmitted from the LD 36 by the PD 37, converts the received optical signal into the original image signal, which is the electric signal, and the magnetic field measurement result, and then outputs the converted image signal and the magnetic field measurement result to the navigation device 12.

The navigation device 12 outputs the image signal for generating the observation image 41 input from the light source device 11 to the processor device 14. In addition, the navigation device 12 controls the drive of the magnetic field generator 13 described below, detects the insertion state of the insertion part 17 in the body of the subject H, and outputs a detection result to the processor device 14 as the information for generating the insertion part shape image 42.

As described above, the endoscope 10 in the present embodiment is one-connector type having one connector 34 connected to the light source device 11. The endoscope 10 is connected to each of the processor device 14 and the navigation device 12 so as to be capable of communication via the light source device 11 to which the connector 34 is connected.

The magnetic field generator 13 has a plurality of generation coils 39 corresponding to a plurality of magnetic field generation elements. Each generation coil 39 includes, for example, an X-axis coil, a Y-axis coil, and a Z-axis coil that generate, by applying a drive current, an alternating current magnetic field (alternating current magnetic field) in directions corresponding to XYZ coordinate axes of an orthogonal coordinate system XYZ, respectively. of communication via the light source device 11 to which the connector 34 is connected.

The magnetic field generator 13 has a plurality of generation coils 39 corresponding to a plurality of magnetic field generation elements. Each generation coil 39 includes, for example, an X-axis coil, a Y-axis coil, and a Z-axis coil that generate, by applying a drive current, an alternating current magnetic field in directions corresponding to XYZ coordinate axes of an orthogonal coordinate system XYZ, respectively. Each generation coil 39 generates the magnetic field MF having the same frequency. The generation coils 39 generate the magnetic fields MF at different timings from each other under a control of the navigation device 12, which will be described in detail below.

### <Endoscope>

Fig. 3 is a block diagram showing an electric configuration of the endoscope system 9. As shown in Fig. 3, the endoscope 10 includes the light guide 33, an irradiation lens 45, the illumination window 46, the observation window 47, the imaging apparatus 48, a magnetic field detection circuit 49, an integrated control circuit 50, the signal cable 32, the LD 36, and the fluid tube and the cleaning nozzle (which are not shown).

The light guide 33 is, for example, a large-diameter optical fiber or a bundle fiber. An incident end of the light guide 33 is inserted into the light source device 11 via the connector 34. The light guide 33 is inserted into the connector 34, the universal cord 19, and the operating part 18, and the insertion part 17, and an emission end faces the irradiation lens 45 provided in the distal end part 23 of the insertion part 17. As a result, the illumination light supplied from the light source device 11 to the incident end of the light guide 33 is emitted to the observation site from the irradiation lens 45 through the illumination window 46 provided on the distal end surface of the distal end part 23. Moreover, the illumination light reflected by the observation site is incident on an imaging surface of the imaging apparatus 48 as image light of the observation site through the observation window 47 provided on the distal end surface of the distal end part 23.

Note that one end side of the fluid tube described above is connected to the air/water supply device through the connector 34 and the light source device 11, and the other end side of the fluid tube is connected to an air/water supply nozzle (not shown) provided on the distal end surface of the distal end part 23 through the insertion part 17 and the like. As a result, the gas or the water supplied from the air/water supply device is injected into the observation window 47 from the air/water supply nozzle to clean the observation window 47.

The imaging apparatus 48 includes a condenser lens 52 and an imaging element 53. which is an observation target. More specifically, the imaging element 53 images the image light of the observation site imaged on the imaging surface (converts the image light into the electric signal), and outputs the image signal of the observation site to the integrated control circuit 50.

In addition, the imaging element 53 is provided with an oscillation unit 53a that outputs a reference signal (clock signal), such as a crystal oscillator, and the imaging element 53 outputs the image signal constituting the motion picture with the reference signal oscillated from the oscillation unit 53a as a reference. An interval of the reference signal defines a frame rate. The frame rate is, for example, 30 frames per second (fps).

The magnetic field detection circuit 49 is electrically connected to each detection coil 25 in the insertion part 17. The magnetic field detection circuit 49 outputs magnetic field measurement data 55 including the magnetic field measurement result of each detection coil 25 depending on the magnetic field MF generated from the generation coil 39 of the magnetic field generator 13 to the integrated control circuit 50.

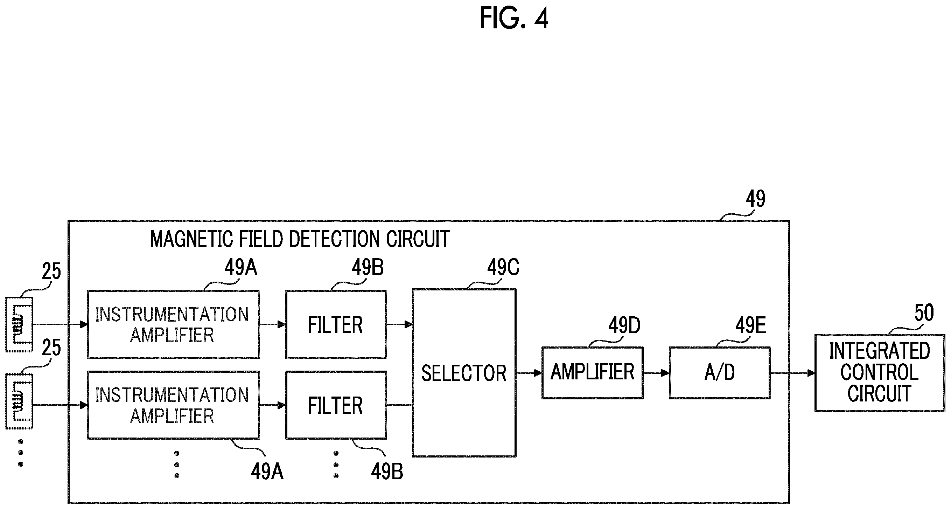

Specifically, as shown in Fig. 4, the magnetic field detection circuit 49 includes an instrumentation amplifier 49A, a filter 49B, a selector 49C, an amplifier 49D, and an analog/digital (A/D) conversion circuit 49E. The instrumentation amplifier 49A is provided for each detection coil 25, and an input terminal thereof is connected to each detection coil 25. The instrumentation amplifier 49A amplifies a weak detection signal output by each detection coil 25 and outputs the amplified detection signal as a voltage corresponding to the value of the detection signal. An output terminal of each instrumentation amplifier 49A is connected to the filter 49B.

The filter 49B is a filter, such as a low pass filter (LPF) or a band pass filter (BPF), and is provided for each instrumentation amplifier 49A, and an input terminal thereof is connected to each instrumentation amplifier 49A. The filter 49B passes a signal of a preset frequency band among the signals input from the instrumentation amplifier 49A. An output terminal of each filter 49B is connected to the selector 49C.

The selector 49C selects the filter 49B, which is a readout target, from the filters 49B. Among a plurality of the filters 49B, the voltage from the filter 49B selected by the selector 49C is input to the amplifier 49D via the selector 49C. The selector 49C sequentially selects each filter 49B based on a timing signal input from a magnetic field measurement control unit 58. Therefore, the voltages corresponding to the detection signals of the plurality of detection coils 25 are sequentially read out.

The amplifier 49D amplifies the voltage output from the selector 49C. More specifically, the amplifier 49D is, for example, an operational amplifier, and amplifies a voltage value of a difference between an input voltage input from the selector 49C and a reference voltage input as a reference and output the amplified voltage value. The A/D conversion circuit 49E converts an output voltage of the amplifier 49D into a digital signal. The A/D conversion circuit 49E outputs a value of this digital signal as the magnetic field measurement result of each detection coil 25 (value corresponding to the magnitude of the induced current indicating the intensity of the magnetic field MF). By sequentially selecting each detection coil 25 by the selector 49C, the A/D conversion circuit 49E outputs the magnetic field measurement data 55 including the respective magnetic field measurement results based on the detection signals of all of the detection coils 25 to the integrated control circuit 50.

The integrated control circuit 50 configured to include arithmetic circuits including various central processing units (CPU) and various memories, and controls the operations of the units of the endoscope 10 in an integrated manner. The integrated control circuit 50 functions as a signal processing unit 57, a magnetic field measurement control unit 58, and an image signal output unit 59 by executing a control program stored in a memory (not shown). The magnetic field detection circuit 49 and the magnetic field measurement control unit 58 correspond to a magnetic field measurement unit. The magnetic field measurement unit measures a plurality of the magnetic fields MF originating from the generation coils 39 corresponding to the plurality of magnetic field generation elements based on the detection signals output by the detection coils 25, and outputs the magnetic field measurement result for each magnetic field MF. The magnetic field measurement unit and the detection coil 25 are combined to configure the magnetic field measurement device.

The signal processing unit 57 performs various signal processes on the image signals sequentially output from the imaging element 53. The signal process includes, for example, an analog signal process, such as a sampling two correlation pile process and a signal amplification process, and an A/D conversion process of converting an analog signal into a digital signal after the analog signal process. The image signal after the signal process is performed is called a frame image signal 61. The signal processing unit 57 outputs the frame image signal 61 to the image signal output unit 59 depending on the frame rate. The frame image signal 61 is used as motion picture data of the observation site. As described above, a plurality of the frame image signals 61 are the image signals that are acquired by the imaging element 53 executing motion picture imaging and are output at time intervals depending on the frame rate.

The magnetic field measurement control unit 58 acquires the magnetic field measurement data 55 including a plurality of the magnetic field measurement results of the detection coils 25 via the magnetic field detection circuit 49, and outputs the acquired magnetic field measurement data 55 to the image signal output unit 59.

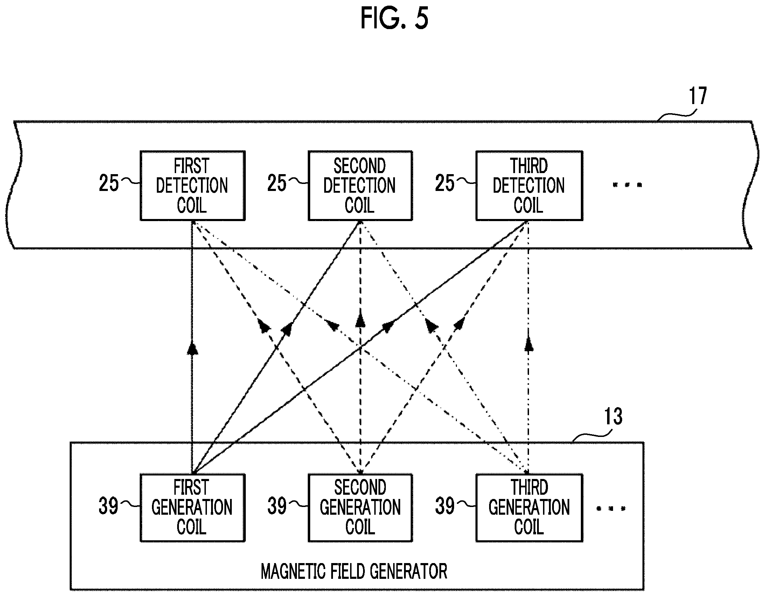

As shown in Fig. 5, even in a case in which the intensity of the magnetic field generated by each generation coil 39 is the same, for example, the magnetic field measurement result of each detection coil 25 is changed depending on a distance and a direction between each generation coil 39 that generates the magnetic field MF and each detection coil 25. For example, the first generation coil 39 shown in Fig. 5 has different distance and direction from each of the first to third detection coils 25, as shown by a solid line. Therefore, the magnetic field measurement results of the first to third detection coils 25 for the magnetic field MF generated by one first generation coil 39 are different. A relationship between each of the second generation coil 39 and the third generation coil 39 and each of the first to third detection coils 25 is the same.

In addition, on the contrary, even in a case in which the intensity of the magnetic field MF generated by each of the first to third generation coils 39 is the same, the magnetic field measurement result of one first detection coil 25 for the magnetic field MF of each generation coil 39 is different. Here, for example, a case is considered in which the first to third generation coils 39 are the X-axis coil, the Y-axis coil, and the Z-axis coil, respectively. In this case, a three-dimension coordinate position of the first detection coil 25 corresponding to the XYZ coordinate axes can be detected based on the magnetic field measurement result of one first detection coil 25 for the magnetic field MF of each of the X-axis coil, the Y-axis coil, and the Z-axis coil. The same applies to the second detection coil 25 and the third detection coil 25. In a case in which the three-dimension coordinate positions of the detection coils 25 provided in the insertion part 17 at preset intervals can be detected, the shape of the insertion part 17 can be detected. In addition, in a case in which the three-dimension coordinate positions of the detection coils 25 disposed in the vicinity of the distal end part 23 can be detected, the position of the distal end part 23 can be detected.

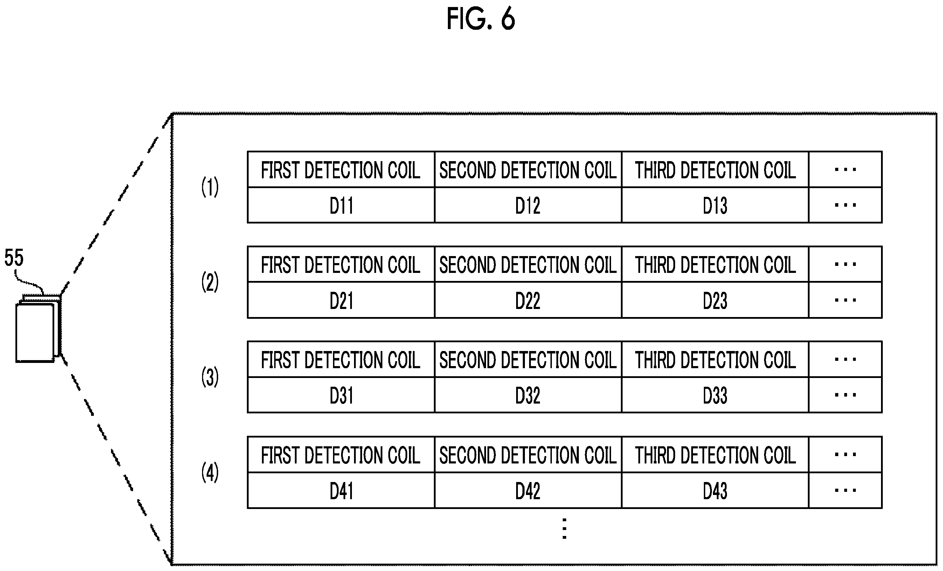

Fig. 6 is an explanatory diagram for describing an example of the magnetic field measurement data 55 acquired by the magnetic field measurement control unit 58. The magnetic field measurement control unit 58 detects the plurality of magnetic fields MF generated by the plurality of generation coils 39 by the plurality of detection coils 25 and acquires the magnetic field measurement data 55 including the plurality of magnetic field measurement results output from the detection coils 25.

In Fig. 6, (1) to (4) are data strings showing the magnetic field measurement results of the plurality of detection coils 25 with respect to the magnetic fields MF generated by the generation coils 39, respectively. For example, "D11" is a magnetic field measurement result in which the magnetic field MF generated by the first driven generation coil 39 is detected by the "first detection coil". "D12" is a magnetic field measurement result in which the magnetic field MF generated by the first driven generation coil 39 is detected by the "second detection coil". Similarly, "D42" is a magnetic field measurement result in which the magnetic field MF generated by the fourth driven generation coil 39 is detected by the "second detection coil". "D43" is a magnetic field measurement result in which the magnetic field MF generated by the fourth driven generation coil 39 is detected by the "third detection coil".

The magnetic field measurement control unit 58 sequentially acquires the magnetic field measurement results of the detection coils 25 while synchronizing with a magnetic field generation timing of each generation coil 39 in the magnetic field generator 13. The magnetic field measurement control unit 58 acquires, for example, the magnetic field measurement results of all of the detection coils 25 for the magnetic fields MF of all of the generation coils 39 in one magnetic field measurement period defined by a synchronization signal described below. As a result, the magnetic field measurement control unit 58 acquires the magnetic field measurement data 55 including the plurality of magnetic field measurement results relating to all of combinations of the generation coils 39 and the detection coils 25 in one magnetic field measurement period. The magnetic field measurement period corresponds to a predetermined measurement unit time of the magnetic field. In the present embodiment, the magnetic field measurement period is an interval of the reference signal that defines the frame rate of the imaging element 53.

For example, in a case in which 9 generation coils 39 are provided in the magnetic field generator 13 and 17 detection coils 25 are provided in the insertion part 17, 17 magnetic field measurement results are obtained for each generation coil 39. Therefore, the magnetic field measurement control unit 58 acquires the magnetic field measurement data 55 including a total of 9 × 17 = 153 magnetic field measurement results in one magnetic field measurement period. The magnetic field measurement data 55 including the plurality of magnetic field measurement results relating to all of such combinations is referred to as total magnetic field measurement data. In the present embodiment, unless otherwise specified, the magnetic field measurement data 55 shall include the total magnetic field measurement data.

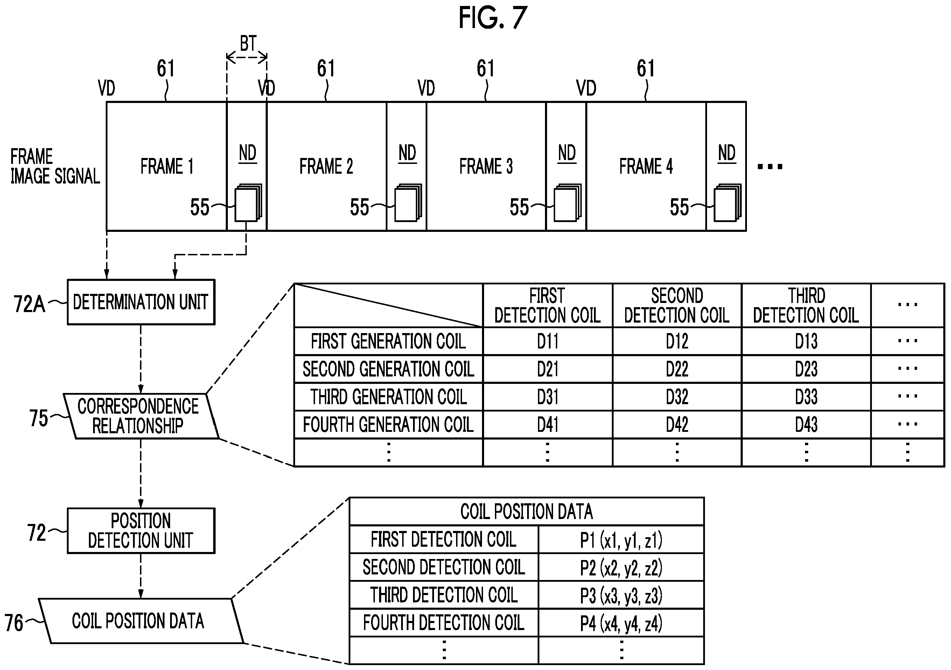

As shown in Fig. 7, the image signal output unit 59 adds a frame start signal VD to each of the plurality of frame image signals 61 sequentially input from the signal processing unit 57, and outputs the frame image signal 61. In Fig. 7, "frame 1", "frame 2", "frame 3" ... are frame numbers indicating output order of the plurality of frame image signals 61, which are shown for convenience. The frame start signal VD is a vertical synchronization signal, for example.

Further, the image signal output unit 59 adds the magnetic field measurement data 55 to the frame image signal 61, and outputs the frame image signal 61. That is, the frame start signal VD and the magnetic field measurement data 55 are included in all of the frame image signals 61 output by the image signal output unit 59. As shown in Fig. 7, the magnetic field measurement data 55 is added to a signal invalid region ND between the frame image signals 61, which corresponds to a blanking time BT of the imaging element 53. The blanking time BT is a vertical blanking period, for example. The frame start signal VD is also the signal included in the vertical blanking period of the plurality of frame image signals 61.

Via the signal cable 32, the image signal output unit 59 outputs the frame image signal 61 to the LD 36. The LD 36 transmits the optical signal obtained by converting the frame image signal 61 into the optical signal to the PD 37 of the light source device 11.

As described above, the image signal output unit 59 outputs the frame image signal 61 acquired from the imaging element 53 via the signal processing unit 57 to the outside of the endoscope 10. In addition, by using the frame start signal VD included in the frame image signal 61 as the synchronization signal, the image signal output unit 59 functions as a synchronization signal generation unit.

### <Light Source Device>

The light source device 11 includes an illumination light source 63, the PD 37, a light source control unit 64, and a communication interface 65. The illumination light source 63 is, for example, a semiconductor light source, such as the LD or a light emitting diode (LED), and is a white light source which emits white light having a wavelength range from a red region to a blue region as the illumination light. Note that, as the illumination light source 63, in addition to the white light source, a special light source that emits special light, such as purple light and infrared light, may be used. The illumination light emitted from the illumination light source 63 is incident on the incident end of the light guide 33.

The PD 37 receives the optical signal transmitted from the LD 36. The PD 37 converts the frame image signal 61 received in the form of an optical signal into the form of the original electric signal. The converted frame image signal 61 from the PD 37 is input to the light source control unit 64.

The light source control unit 64 is configured to include various arithmetic circuits including the CPU and various memories, and controls the operation of each unit of the light source device 11, such as the illumination light source 63. In addition, the converted frame image signal 61 from the PD 37 is output to the navigation device 12 via the light source control unit 64 and the communication interface 65.

### <Navigation Device>

The navigation device 12 includes an image signal acquisition unit 68, an insertion state detection unit 69, a timing generator (hereinafter referred to as "TG") 70, a magnetic field generation control unit 71, and a display output unit 74. Each unit of the navigation device 12 is configured by various arithmetic circuits (not shown) including one or a plurality of CPUs, and is operated by executing a control program stored in a memory (not shown).

Via the communication interface 65, the image signal acquisition unit 68 acquires the frame image signal 61 from the light source control unit 64. Moreover, the image signal acquisition unit 68 outputs the acquired frame image signal 61 to the display output unit 74.

In addition, the image signal acquisition unit 68 extracts the frame start signal VD and the magnetic field measurement data 55 included in the frame image signal 61, and outputs the extracted frame start signal VD and the magnetic field measurement data 55 to the insertion state detection unit 69. In addition, the image signal acquisition unit 68 outputs the frame start signal VD extracted from the frame image signal 61 to the TG 70.

Based on the frame start signal VD input from the image signal acquisition unit 68, the TG 70 outputs a clock signal for control of selectively switching each generation coil 39 to the magnetic field generation control unit 71. The magnetic field generation control unit 71 controls a start timing of the magnetic field generation of each generation coil 39 based on the clock signal input from the TG 70. Further, the magnetic field generation control unit 71 also controls the order of the magnetic field generation of each generation coil 39 in each magnetic field measurement period. Details of the control by the magnetic field generation control unit 71 will be described below.

The insertion state detection unit 69 detects the insertion state of the insertion part 17 of the endoscope 10 inserted into the body of the subject H based on the frame start signal VD and the magnetic field measurement data 55 acquired from the image signal acquisition unit 68. The insertion state detection unit 69 includes a position detection unit 72 and an insertion part shape detection unit 73.

The position detection unit 72 detects a position of each detection coil 25 based on the frame start signal VD and the magnetic field measurement data 55. The position detection unit 72 includes a determination unit 72A.

As shown in Fig. 7, the determination unit 72A determines the plurality of magnetic field measurement results included in the magnetic field measurement data 55 with reference to a correspondence relationship 75. The correspondence relationship 75 is information indicating storage order of the plurality of magnetic field measurement results corresponding to a plurality of combinations of the generation coils 39 and the detection coils 25 included in the magnetic field measurement data 55. The determination unit 72A determines which combination of each generation coil 39 and each detection coil 25 corresponds to each magnetic field measurement result included in the magnetic field measurement data 55 based on the correspondence relationship 75.

Specifically, as will be described below, in the magnetic field measurement, the generation order in which the generation coils 39 generate the magnetic field MF with the frame start signal VD as a reference and the acquisition order of the magnetic field measurement results of the detection coils 25 for the magnetic field MF of one generation coil 39 are decided for each magnetic field measurement period. The plurality of magnetic field measurement results corresponding to the combinations of the generation coils 39 and the detection coils 25 are stored in the magnetic field measurement data 55 depending on the generation order and the acquisition order. Therefore, the determination unit 72A can determine which combination of the plurality of magnetic field measurement results included in the magnetic field measurement data 55 ("D11", "D12", "D13", ...) corresponds to each magnetic field measurement result by referring to the correspondence relationship 75 that defines the storage order with the frame start signal VD as a reference.

The position detection unit 72 detects, as coil position data 76, the position of each detection coil 25, specifically, the three-dimension coordinate position based on the plurality of magnetic field measurement results determined by the determination unit 72A. The coil position data 76 is a relative position with the magnetic field generator 13 as a reference. In Fig. 7, for example, P1 indicates the three-dimension coordinate position (x1, y1, and z1) of the first detection coil 25. The same applies to P2, P3, P4, and the like.

In the present embodiment, the position detection unit 72 detects the position of each detection coil 25 based on the magnetic field measurement data 55 obtained by averaging the magnetic field measurement data 55 of the latest predetermined number (for example, 5) of magnetic field measurement periods. As a result, an influence of a noise can be reduced. Note that, for averaging, the position detection unit 72 stores at least the latest 5 magnetic field measurement data 55 in the storage unit, such as a non-volatile memory, provided in the navigation device 12. In this case, the position detection unit 72 replaces the oldest magnetic field measurement data 55 for each time new magnetic field measurement data 55 input.

The position detection unit 72 outputs the coil position data 76 to the insertion part shape detection unit 73. The insertion part shape detection unit 73 detects the shape of the insertion part 17 in the body of the subject H based on the coil position data 76 input from the position detection unit 72.

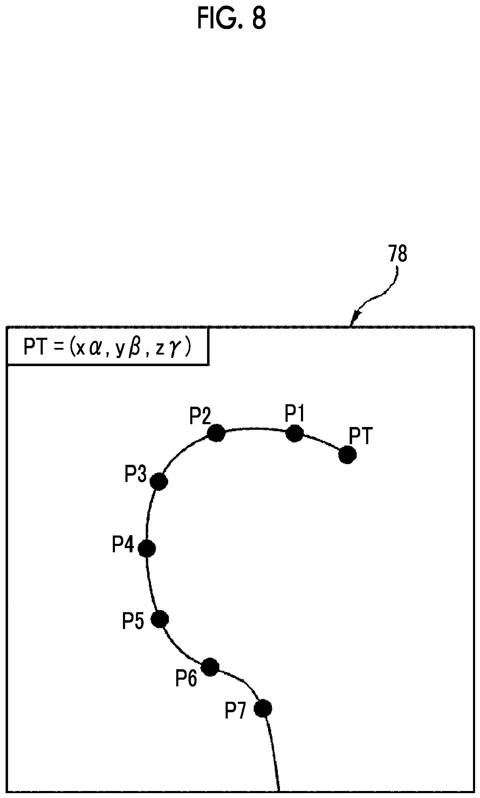

Fig. 8 is an explanatory diagram for describing an example of a shape detection process of the insertion part 17 by the insertion part shape detection unit 73. As shown in Fig. 8, the insertion part shape detection unit 73 performs an interpolation process of performing interpolation on each position with a curve based on the position (P1, P2, ...) of each detection coil 25 indicated by the coil position data 76, and generates insertion part shape data 78 showing the shape of the insertion part 17. The insertion part shape data 78 includes a distal end position PT of the distal end part 23 of the insertion part 17.

The insertion part shape detection unit 73 outputs the insertion part shape data 78 to the display output unit 74. The insertion state detection unit 69 repeatedly performs a determination process by the determination unit 72A, a position detection process of detecting the coil position data 76 of each detection coil 25, a shape detection process of the insertion part 17, and an output process of the insertion part shape data 78 each time the image signal acquisition unit 68 acquires new frame image signal 61.

The display output unit 74 outputs the frame image signal 61 input from the image signal acquisition unit 68 and the insertion part shape data 78 input from the insertion state detection unit 69 to the processor device 14 via communication interfaces 80A and 80B. In this case, the display output unit 74 associates the frame image signal 61 with the insertion part shape data 78 that corresponds in time with the frame image signal 61, and outputs the data to the processor device 14.

### <Processor Device>

The processor device 14 has a display input unit 82 and a display control unit 83. The display input unit 82 sequentially outputs, to the display control unit 83, the data of the frame image signal 61 and the insertion part shape data 78, which are sequentially input from the display output unit 74 via the communication interfaces 80A and 80B.

The display control unit 83 receives the input of the frame image signal 61 and the insertion part shape data 78 from the display input unit 82, and displays the observation image 41 (motion picture) based on the frame image signal 61 and the insertion part shape image 42 based on the insertion part shape data 78 on the monitor 15. As described above, the frame image signal 61 is transmitted from the endoscope 10 to the processor device 14 via the light source device 11 and the navigation device 12. Moreover, the processor device 14 performs image processing on the frame image signal 61 acquired from the endoscope 10 to generate the observation image 41 which is an example of a display image.

### <Control of Driving Order of Generation Coils>

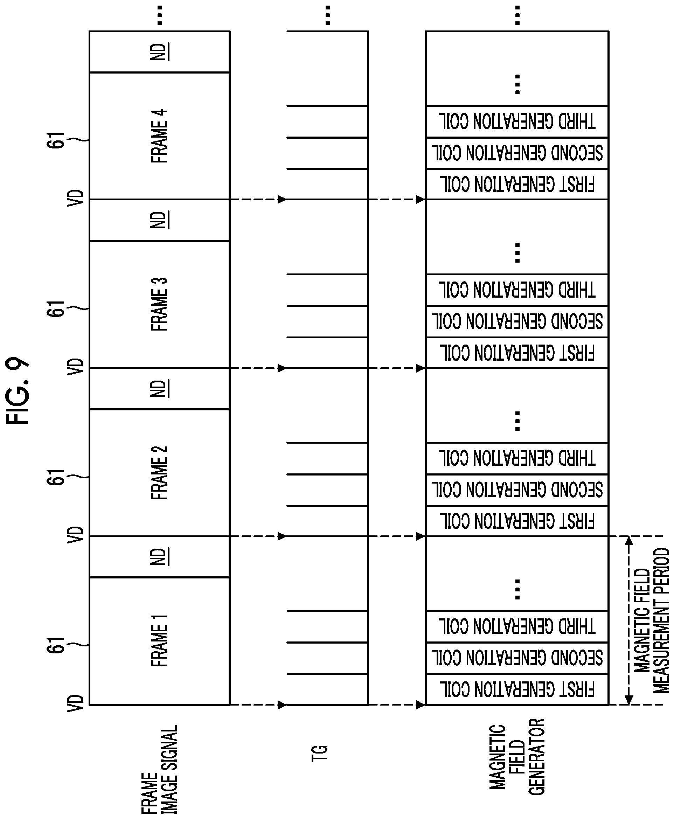

First, with reference to Figs. 9 to 11, a problem in a case in which the driving order of the generation coils 39 within each magnetic field measurement period is the same order as in the related art will be described.

As shown in Fig. 9, each magnetic field measurement period is based on the clock signal input from the TG 70 to the magnetic field generation control unit 71, which corresponds to the frame start signal VD. In addition, within each magnetic field measurement period, the clock signal is input from the TG 70 to the magnetic field generation control unit 71 depending on the preset frequency. The frequency is set to a frequency at which the generation coil 39 that generates the magnetic field can make a round during each magnetic field measurement period, that is, between two consecutive frame start signals VD. In addition, the frequency is also set in advance in the magnetic field measurement control unit 58, and the generation of the magnetic field from each generation coil 39 and the measurement of the magnetic field by the magnetic field measurement control unit 58 are performed in synchronization with each other.

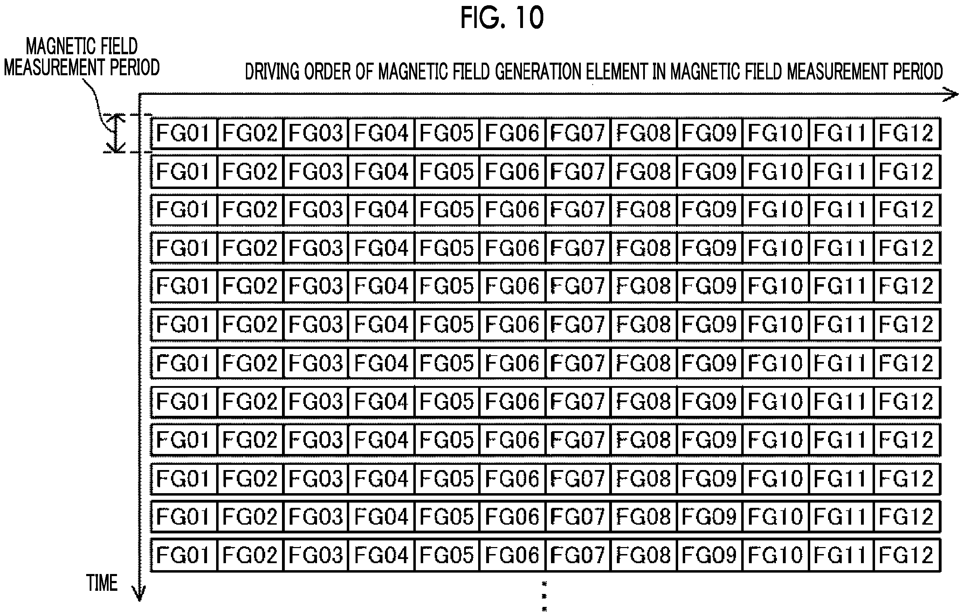

In the examples of Figs. 9 and 10, the generation coils 39 are driven in the same order in response to the clock signal input from the TG 70 to the magnetic field generation control unit 71 within each magnetic field measurement period. In Fig. 10, "FG" means the generation coil, and the number after "FG" means the number that distinguishes the generation coil 39. For example, "FG1" in Fig. 10 corresponds to a "first generation coil" in Fig. 9. Fig. 10 shows an example in which the driving order of the 12 generation coils 39 within each magnetic field measurement period is the same order. In this example, the driving period of each generation coil 39 is fixed.

For example, a case will be considered in which a periodic noise, such as a noise caused by a power source, affects the magnetic field generated by the generation coil 39. In this case, in response to the clock signal input from the TG 70 to the magnetic field generation control unit 71 within each magnetic field measurement period. In Fig. 10, "FG" means the generation coil, and the number after "FG" means the number that distinguishes the generation coil 39. For example, "FG01" in Fig. 10 corresponds to a "first generation coil" in Fig. 9. Fig. 10 shows an example in which the driving order of the 12 generation coils 39 within each magnetic field measurement period is the same order. In this example, the driving period of each generation coil 39 is fixed.

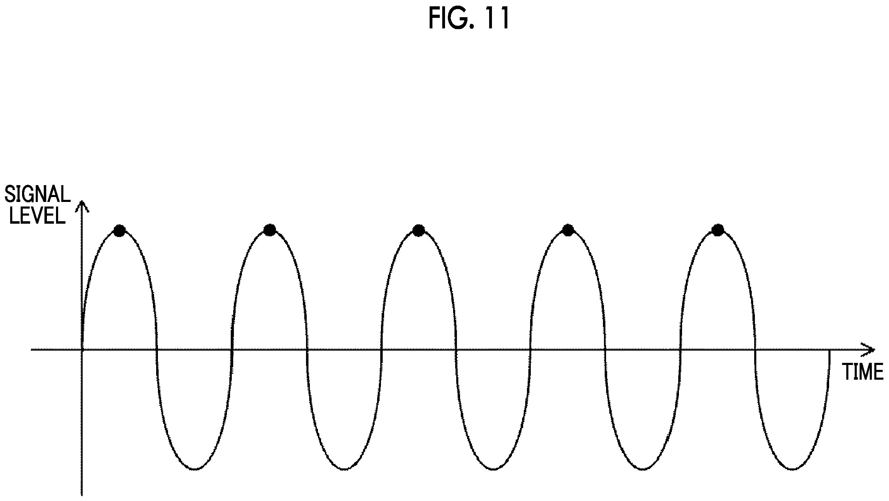

For example, a case will be considered in which a periodic noise, such as a noise caused by a power source, affects the magnetic field generated by the generation coil 39. In this case, in a case in which the driving period of the generation coil 39 and the noise period are substantially the same, as shown in Fig. 11, the noise of a relatively high signal level is superimposed on the magnetic field generated from the specific generation coil 39. A vertical axis of Fig. 11 represents a signal level of the noise, and a horizontal axis represents a time. In addition, a circle in Fig. 11 represents a timing at which the specific generation coil 39 is driven. Fig. 11 shows an example in which the noise of the maximum signal level is superimposed on the magnetic field generated from the specific generation coil 39.

In this case, relatively large noise is superimposed on a measurement result of the magnetic field generated from the specific generation coil 39. Therefore, in a case in which the position detection unit 72 detects the position of the generation coil 39 by using the magnetic field measurement data 55 including the measurement result of the magnetic field, it is not possible to detect the position of the specific generation coil 39 with high accuracy. As a result, it is not possible to detect the shape of the insertion part 17 of the endoscope 10 with high accuracy.

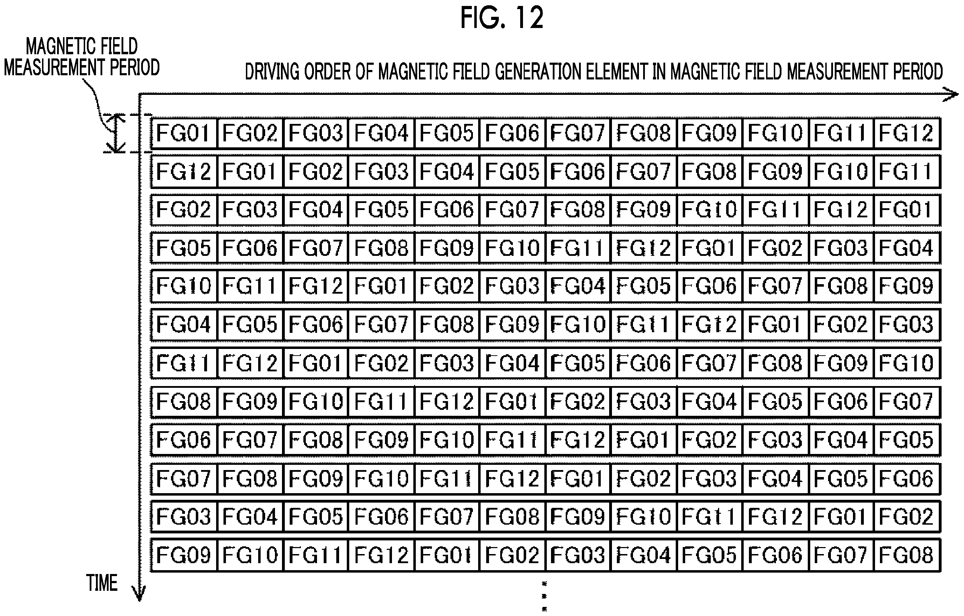

Therefore, as shown in Fig. 12 as an example, the magnetic field generation control unit 71 according to the present embodiment performs a control of varying the driving order of the generation coils 39 within the magnetic field measurement period between the plurality of magnetic field measurement periods. In the present embodiment, the magnetic field generation control unit 71 randomly decides the generation coil 39 to be driven at the beginning of each magnetic field measurement period, and drives the generation coil 39 in the numerical order for distinguishing the generation coil 39 from the generation coil 39. Note that, as long as the driving order of the generation coils 39 within the magnetic field measurement period is varied between the plurality of magnetic field measurement periods, there is no particular limitation on how to vary the driving order. For example, the driving order may be randomly varied.

In addition, as described above, the position detection unit 72 detects the position of each detection coil 25 based on the magnetic field measurement data 55 obtained by averaging the magnetic field measurement data 55 of the latest predetermined number of the magnetic field measurement periods. Therefore, for example, it is preferable that the magnetic field generation control unit 71 drive each generation coil 39 in the driving order in which a driving interval between the magnetic field measurement periods of each generation coil 39 is not fixed within the magnetic field measurement period of the number of averaging targets (for example, 5) by the position detection unit 72. That is, it is preferable that the driving order of each generation coil 39 be varied within the five magnetic field measurement periods.

In the present embodiment, for example, the storage unit, such as the non-volatile memory, included in the navigation device 12 stores rule information indicating by what rule the driving order of the generation coils 39 is varied. Moreover, the magnetic field generation control unit 71 performs the control of varying the driving order of the generation coils 39 within the magnetic field measurement period between the plurality of magnetic field measurement periods according to the rule information.

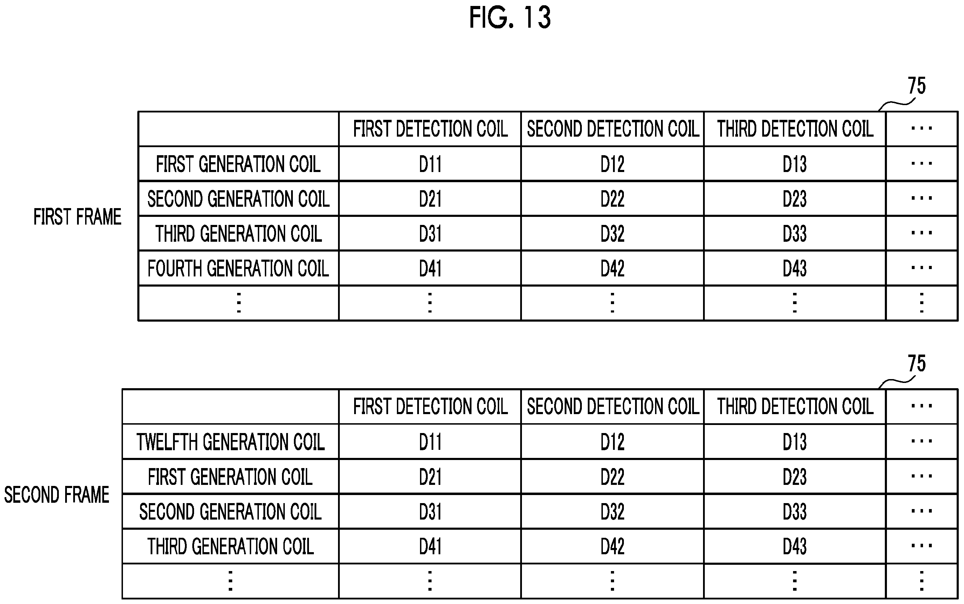

The determination unit 72A can specify the correspondence relationship 75 described above from information indicating a magnetic field measurement period number after the start of the magnetic field measurement (that is, a frame number) and the rule information described above. Fig. 13 shows the correspondence relationship 75 of a first frame and the correspondence relationship 75 of a second frame in a case in which the generation coil 39 is driven in the driving order shown in Fig. 12. In Fig. 13, the "first generation coil" to "twelfth generation coil" have one-to-one correspondence relationship with "FG01" to "FG 12" in Fig. 12. In the examples of Figs. 12 and 13, the determination unit 72A specifies that in the first frame, the first magnetic field measurement result of the magnetic field measurement data 55 is based on the magnetic field generated by "FG01", and in the second frame, the first magnetic field measurement result of the magnetic field measurement data 55 is based on the magnetic field generated by "FG12".

### <Operation of Endoscope System>

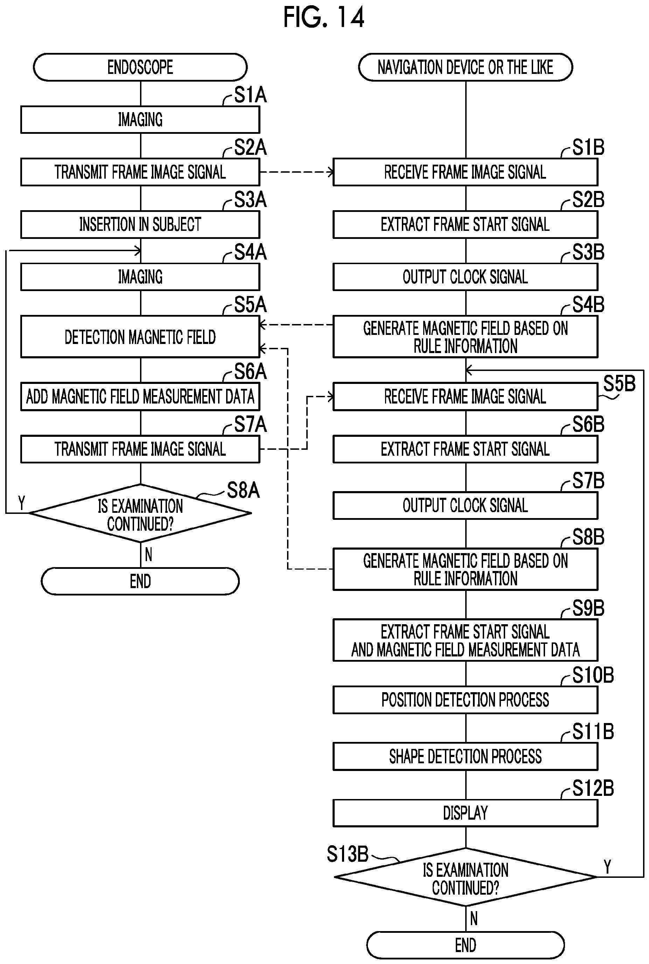

Next, an operation of the endoscope system 9 according to the present embodiment will be described with reference to Fig. 14. Note that Fig. 14 is a flowchart showing an example of a display process of the observation image 41 and the insertion part shape image 42. The display process is executed, for example, after the power switch of the endoscope

In step S2A, the LD 36 transmits the optical signal obtained by converting the frame image signal 61 input from the image signal output unit 59 into the optical signal to the PD 37 of the light source device 11.

In step S1B, the PD 37 of the light source device 11 receives the optical signal transmitted from the LD 36. In addition, the PD 37 converts the frame image signal 61 received in a form of the optical signal into a form of the original electric signal. The converted frame image signal 61 from the PD 37 is output to the navigation device 12 via the light source control unit 64 and the communication interface 65.

In step S2B, the image signal acquisition unit 68 acquires the frame image signal 61 from the light source control unit 64 via the communication interface 65 and extracts the frame start signal VD from the acquired frame image signal 61. Moreover, the image signal acquisition unit 68 outputs the extracted frame start signal VD to the TG 70.

In step S3B, the TG 70 outputs the clock signal for control of switching each generation coil 39 to the magnetic field generation control unit 71 based on the frame start signal VD from the image signal acquisition unit 68. In step S4B, the magnetic field generation control unit 71 drives the generation coils 39 at different timings based on the clock signal input from the TG 70. As a result, the magnetic fields are generated from the generation coils 39 at different timings. In this case, the magnetic field generation control unit 71 drives each generation coil 39 in the order defined as the first frame based on the rule information described above. By the processes up to step S4B described above, the activation of the endoscope system 9 is completed.

Next, in step S3A, the insertion part 17 of the endoscope 10 is inserted into the subject H by the operator OP, and imaging of the observation site in the subject H is started. The illumination light supplied from the illumination light source 63 of the light source device 11 is emitted from the illumination window 46 to the observation site through the light guide 33 and the irradiation lens 45. In step S4A, the imaging element 53 images the image light of the observation site incident through the observation window 47 and the condenser lens 52. As a result, the imaging element 53 outputs the image signal to the integrated control circuit 50 with reference to the reference signal oscillated from the oscillation unit 53a. The image signal input from the imaging element 53 to the integrated control circuit 50 is output to the image signal output unit 59 as the frame image signal 61 after various signal processes are performed by the signal processing unit 57 of the integrated control circuit 50.

In step S5A, the magnetic field measurement control unit 58 controls the magnetic field detection circuit 49 at the frequency corresponding to the clock signal of the TG 70 based on the reference signal oscillated from the oscillation unit 53a, and repeatedly acquires the magnetic field measurement results detected by the detection coils 25. That is, the magnetic field measurement control unit 58 acquires the magnetic field measurement result, for each generation coil 39, detected by each detection coil 25 in synchronization with the switching of the generation coil 39. Moreover, the magnetic field measurement control unit 58 outputs the magnetic field measurement data 55 including all of the acquired magnetic field measurement results to the image signal output unit 59 in synchronization with the output of the frame image signal 61 from the signal processing unit 57 to the image signal output unit 59.

In step S6A, the image signal output unit 59 adds the frame start signal VD to the frame image signal 61 input from the signal processing unit 57, and adds the magnetic field measurement data 55 input from the magnetic field measurement control unit 58 to the signal invalid region ND. In step S7A, the image signal output unit 59 outputs, to the LD 36, the frame image signal 61 to which the frame start signal VD and the magnetic field measurement data 55 are added. The LD 36 transmits the optical signal obtained by converting the frame image signal 61 input from the image signal output unit 59 into the optical signal to the PD 37 of the light source device 11.

In step S5B to step S8B, the same processes as in step S1B to step S4B are executed. In this case, in step S8B, each time step S8B is executed, the driving order of the generation coil 39 is decided from the information indicating the frame number and the rule information described above. Therefore, the generation coil 39 is driven in the varied driving orders for each frame.

In step S9B, the image signal acquisition unit 68 extracts the frame start signal VD and the magnetic field measurement data 55 included in the frame image signal 61 acquired in step S6B, and outputs the extracted frame start signal VD and the magnetic field measurement data 55 to the insertion state detection unit 69.

In step S10B, the position detection unit 72 detects the position of each detection coil 25 based on the frame start signal VD and the magnetic field measurement data 55 input from the image signal acquisition unit 68, as described above. In this case, the correspondence relationship 75 used by the determination unit 72A is specified from the information indicating the frame number and the rule information described above each time step S10B is executed, as described above. Moreover, the position detection unit 72 outputs the coil position data 76 indicating the detected position of each detection coil 25 to the insertion part shape detection unit 73.

In step S 11B, as described above, the insertion part shape detection unit 73 detects the shape of the insertion part 17 in the body of the subject H based on the coil position data 76 input from the position detection unit 72, and generates the insertion part shape data 78 indicating the shape of the insertion part 17.

In step S12B, the display output unit 74 outputs the frame image signal 61 acquired by the image signal acquisition unit 68 in step S6B and the insertion part shape data 78 generated by the insertion part shape detection unit 73 in step S 11B to the processor device 14 via the communication interfaces 80A and 80B. The frame image signal 61 and the insertion part shape data 78, which are input from the display output unit 74 to the display input unit 82 of the processor device 14, are output to the monitor 15 by the display control unit 83. As a result, the observation image 41 based on the frame image signal 61 and the insertion part shape image 42 based on the insertion part shape data 78 are displayed on the monitor 15.

The processes of step S4A to step S7A and the processes of step S5B to step S12B described above are repeatedly performed until the endoscopic examination is completed (steps S8A and S 13B). As a result, the observation image 41 and the insertion part shape image 42 are updated and displayed on the monitor 15 depending on the preset frame rate.

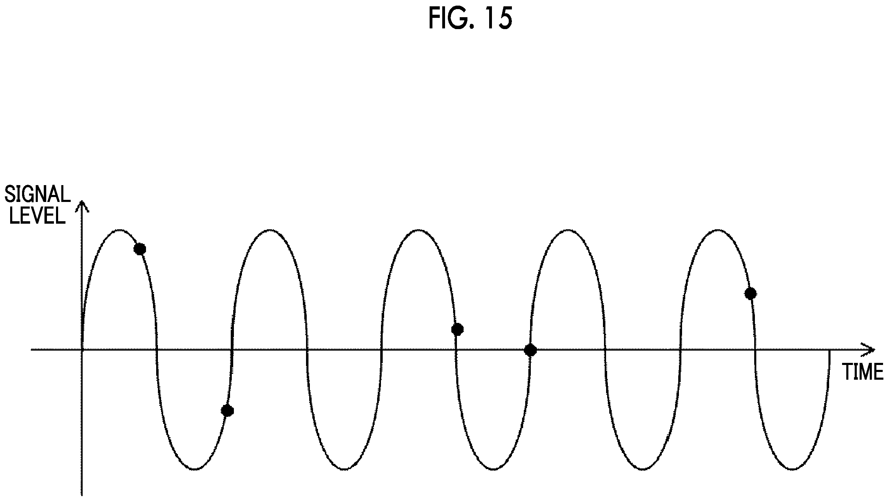

As described above, according to the present embodiment, the driving order of the generation coil 39 within the magnetic field measurement period is varied between the plurality of magnetic field measurement periods. Therefore, for example, as shown in Fig. 15, the driving period of each generation coil 39 is not fixed. A vertical axis of Fig. 15 represents the signal level of the noise, and a horizontal axis represents the time. In addition, a circle in Fig. 15 represents the timing at which one generation coil 39 is driven. As a result, the signal level of the noise superimposed on the magnetic field generated from the generation coil 39 is not fixed, so that it is possible to suppress superimposing of the noise of a relatively high signal level on the magnetic field generated from the specific generation coil 39. As a result, it is possible to detect the insertion state of the insertion part of the endoscope with high accuracy.

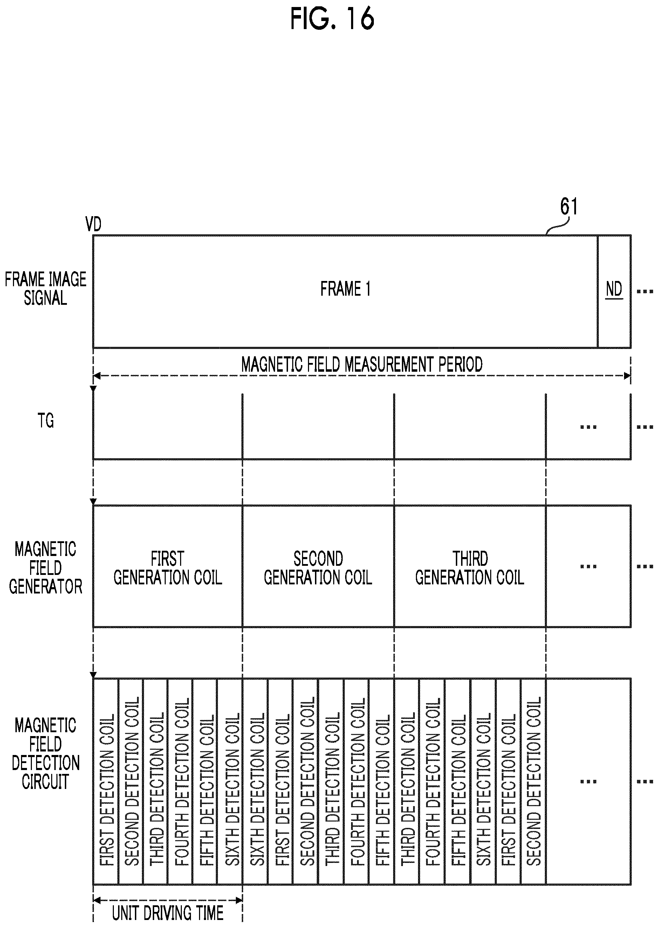

Note that, in the embodiment described above, the case has been described in which the order of acquiring the measurement result of the magnetic field generated within the unit driving time in which each generation coil 39 is driven from the plurality of detection coils 25 within the magnetic field measurement period is the same for each unit driving time. However, the present disclosure is not limited to this. For example, as shown in Fig. 16, a form may be adopted in which the magnetic field measurement control unit 58 performs the control of varying the order of acquiring the measurement result of the magnetic field generated within the unit driving time from the plurality of detection coils 25 within the magnetic field measurement period between the plurality of unit driving times. It is possible to control this order by the selection order of the filter 49B by the selector 49C of the magnetic field detection circuit 49. Therefore, it is possible to reduce the influence of the periodic noise on the measurement result of the magnetic field by the detection coil 25, and as a result, the insertion state of the insertion part of the endoscope can be detected with higher accuracy. Note that Fig. 16 shows an example in a case in which the number of detection coils 25 is six.

In this case, for example, a form is described in which the information indicating by what rule the acquisition order of the measurement result of the magnetic field is varied is stored in the non-volatile memory provided in the integrated control circuit 50. In addition, in this case, the determination unit 72A can specify the correspondence relationship 75 by further using this information.

In addition, in the embodiment described above, the example has been described in which the magnetic field measurement unit including the magnetic field measurement control unit 58 is disposed in the endoscope 10 and the magnetic field generation control unit 71 is disposed in the navigation device 12, on the contrary, the magnetic field generation control unit 71 may be disposed in the endoscope 10 and the magnetic field measurement unit may be disposed in the navigation device 12.

In addition, in the embodiment described above, the case has been described in which the frame start signal VD included in the frame image signal 61 is used as the synchronization signal. However, a signal other than the signal included in the vertical blanking period, for example, a horizontal synchronization signal and the like may be used as the synchronization signal. In addition, the synchronization signal may be embedded in header information of the frame image signal 61 and used. Further, the signal different from the signal included in the frame image signal 61 may be used as the synchronization signal. In this case, a form is adopted in which a transmission route different from that of the frame image signal 61 is provided.

In addition, in the embodiment described above, the case has been described in which the total magnetic field measurement data including the magnetic field measurement results relating to all of the combinations of each generation coil 39 and each detection coil 25 is acquired between two consecutive frame start signals VD, but the present disclosure is not limited to this. For example, total magnetic field measurement data may be acquired in a period in which three or more frame start signals VD are output. The update frequency of the insertion part shape image 42 is decreased as the acquisition period of the total magnetic field measurement data is longer. However, since the insertion part shape image 42 is not required to have a high definition image as compared with the observation image 41, it is permissible in a case of the insertion part shape image 42 even in a case in which the update frequency is decreased as compared with the observation image 41.

In addition, in the embodiment described above, the case has been described in which the frame image signal 61 is converted into the optical signal and transmitted from the endoscope 10 to the light source device 11, but the present disclosure is not limited to this. For example, a form may be adopted in which the endoscope 10 and the light source device 11 are electrically connected via the metal signal line, and the frame image signal 61 is transmitted from the endoscope 10 to the light source device 11 as the electric signal.

In addition, in the embodiment described above, the endoscope 10 used for the examination of the lower digestive tract, such as the large intestine, has been described as an example, but the type and use of the endoscope 10 are not particularly limited. The endoscope 10 for the upper digestive tract may be used. In addition, particularly in a case in which the purpose is to detect the distal end position PT of the insertion part 17, the disclosed technology can be applied to a soft endoscope as well as a rigid endoscope.

In addition, in the embodiment described above, the example has been described in which each generation coil 39 generates the magnetic field MF having the same frequency, but the frequencies of the magnetic fields MF generated by the generation coils 39 may be different. However, in a case in which the frequencies of the magnetic fields MF generated by the generation coils 39 are the same as in the endoscope system 9 according to the embodiment described above, it is not possible to distinguish the plurality of magnetic fields MF from each other. Therefore, in order to distinguish the generation coils 39 of the generation sources of the plurality of magnetic fields MF, there is no choice but to shift the generation timings. Therefore, the technology of the present disclosure is effective in a case in which the frequencies of the magnetic fields MF generated by the generation coils 39 are the same.

In addition, in the embodiment described above, the plurality of generation coils 39 and the plurality of detection coils 25 are provided, but at least one detection coil 25 need only be provided. For example, the technology of the present disclosure may be applied to the endoscope system 9 using the endoscope 10 in which only one detection coil 25 is provided at the distal end part 23 of the insertion part 17. Even in this case, it is possible to present the current position of the distal end part 23.

In addition, in the embodiment described above, for example, as a hardware structure of the processing unit that executes various processes, such as the magnetic field measurement control unit 58 and the magnetic field generation control unit 71, various processors in the following can be used. The various processors include the CPU that is a general-purpose processor executing the software and functioning as the various processing units, as well as a programmable logic device (PLD) that is a processor of which a circuit configuration can be changed after manufacture, such as a field programmable gate array (FPGA) or a dedicated electric circuit that is a processor having a circuit configuration that is designed for exclusive use in order to execute a specific process, such as an application specific integrated circuit (ASIC).

One processing unit may be configured by one of the various processors, or may be a combination of two or more processors of the same type or different types (for example, a combination of a plurality of FPGAs or a combination of a CPU and an FPGA). In this way, as the hardware structure, various processing units are configured by one or more of various processors described above. Stated another way, these processors function as each processing unit in cooperation with the memory built in the processor or the connected memory.

Further, as the hardware structure of these various processors, more specifically, it is possible to use an electric circuit (circuitry) in which circuit elements such as semiconductor elements are combined.

The invention is defined by the appended claims.

## Claims

1. An endoscope system that generates magnetic fields by selectively switching and driving a plurality of magnetic field generation elements (39), and detects an insertion state including at least one of a position or a shape of an insertion part (17) of an endoscope (10) based on measurement results of the magnetic fields by magnetic field detection elements (25) provided in the insertion part (17) of the endoscope (10), the system comprising:
a magnetic field generation control unit (71) that performs a control of varying driving order of the magnetic field generation elements within a predetermined measurement unit time between a plurality of the measurement unit times.

2. The endoscope system according to claim 1, further comprising:
a magnetic field measurement control unit (58) that performs a control of varying order of acquiring, from a plurality of the magnetic field detection elements, the measurement results of the magnetic fields generated within a unit driving time in which one magnetic field generation element is driven within the measurement unit time between a plurality of the unit driving times in a case in which the measurement results of the magnetic fields detected by the plurality of magnetic field detection elements are selectively switched and acquired.

3. The endoscope system according to claim 1 or 2, further comprising:
an insertion state detection unit (69) that detects the insertion state based on a measurement result obtained by averaging the measurement results of the magnetic fields generated by the plurality of magnetic field generation elements for each of a predetermined number of the latest measurement unit times.

4. The endoscope system according to any one of claims 1 to 3,
wherein the magnetic fields generated by the plurality of magnetic field generation elements have the same frequency.

5. An operation method of an endoscope system that generates magnetic fields by selectively switching and driving a plurality of magnetic field generation elements, and detects an insertion state including at least one of a position or a shape of an insertion part of an endoscope based on measurement results of the magnetic fields by magnetic field detection elements (25) provided in the insertion part (17) of the endoscope (10), the method comprising:
a magnetic field generation control step of performing a control of varying driving order of the magnetic field generation elements within a predetermined measurement unit time between a plurality of the measurement unit times.

## Patentansprüche

1. Endoskopsystem, das durch selektives Umschalten und Ansteuern einer Vielzahl von Magnetfelderzeugungselementen (39) Magnetfelder erzeugt und einen Einführungszustand, der zumindest eine von einer Position oder einer Form eines Einführteils (17) eines Endoskops (10) beinhaltet, auf Basis von Messergebnissen der Magnetfelder durch Magnetfelderkennungselemente (25) erfasst, die in dem Einführteil (17) des Endoskops (10) bereitgestellt sind, wobei das System umfasst:
eine Magnetfelderzeugungssteuereinheit (71), die eine Steuerung einer veränderlichen Ansteuerungsreihenfolge der Magnetfelderzeugungselemente innerhalb einer vorbestimmten Messgrößeneinheitszeit zwischen einer Vielzahl der Messgrößeneinheitszeiten durchführt.

2. Endoskopsystem nach Anspruch 1, weiter umfassend:
eine Magnetfeldmessungssteuereinheit (58), die eine Steuerung einer veränderlichen Bezugsreihenfolge, von einer Vielzahl der Magnetfelderfassungselemente, der Messergebnisse der Magnetfelder, die innerhalb einer Einheitsansteuerzeit erzeugt werden, in der ein Magnetfelderzeugungselement innerhalb der Messgrößeneinheitszeit zwischen einer Vielzahl der Einheitsansteuerzeiten angesteuert wird, in einem Fall durchführt, in dem die Messergebnisse der Magnetfelder, die durch die Vielzahl von Magnetfelderfassungselementen erfasst werden, selektiv umgeschaltet und bezogen werden.

3. Endoskopsystem nach Anspruch 1 oder 2, weiter umfassend:
eine Einführungszustandserfassungseinheit (69), die den Einführungszustand auf Basis eines Messergebnisses erfasst, das durch Mitteln der Messergebnisse der Magnetfelder erhalten wird, die durch die Vielzahl von Magnetfelderzeugungselementen für jede einer vorbestimmten Anzahl der neuesten Messgrößeneinheitszeiten erzeugt werden.

4. Endoskopsystem nach einem der Ansprüche 1 bis 3,
wobei die Magnetfelder, die durch die Vielzahl von Magnetfelderzeugungselementen erzeugt werden, dieselbe Frequenz aufweisen.

5. Betriebsverfahren eines Endoskopsystems, das durch selektives Umschalten und Ansteuern einer Vielzahl von Magnetfelderzeugungselementen Magnetfelder erzeugt und einen Einführungszustand, der zumindest eine von einer Position oder einer Form eines Einführteils eines Endoskops beinhaltet, auf Basis von Messergebnissen der Magnetfelder durch Magnetfelderfassungselemente (25) erfasst, die in dem Einführteil (17) des Endoskops (10) bereitgestellt sind, wobei das Verfahren umfasst:
einen Magnetfelderzeugungssteuerungsschritt zum Durchführen einer Steuerung einer veränderlichen Ansteuerungsreihenfolge der Magnetfelderzeugungselemente innerhalb einer vorbestimmten Messgrößeneinheitszeit zwischen einer Vielzahl der Messgrößeneinheitszeiten.

## Revendications

1. Système d'endoscope qui génère des champs magnétiques en commutant et entraînant de manière sélective une pluralité d'éléments de génération de champ magnétique (39), et détecte un état d'insertion incluant au moins l'une d'une position ou d'une forme d'une partie d'insertion (17) d'un endoscope (10) sur la base de résultats de mesure des champs magnétiques par des éléments de détection de champ magnétique (25) disposés dans la partie d'insertion (17) de l'endoscope (10), le système comprenant :
une unité de commande de génération de champ magnétique (71) qui réalise une commande de variation d'ordre d'entraînement des éléments de génération de champ magnétique pendant un temps unitaire de mesure prédéterminé entre une pluralité des temps unitaires de mesure.

2. Système d'endoscope selon la revendication 1, comprenant en outre :
une unité de commande de mesure de champ magnétique (58) qui réalise une commande de variation d'ordre d'acquisition, depuis une pluralité des éléments de détection de champ magnétique, des résultats de mesure des champs magnétiques générés pendant un temps unitaire d'entraînement, dans lequel un élément de génération de champ magnétique est entraîné pendant le temps unitaire de mesure entre une pluralité des temps unitaires d'entraînement dans un cas où les résultats de mesure des champs magnétiques détectés par la pluralité d'éléments de détection de champ magnétique sont commutés et acquis de manière sélective.

3. Système d'endoscope selon la revendication 1 ou 2, comprenant en outre :
une unité de détection d'état d'insertion (69) qui détecte l'état d'insertion sur la base d'un résultat de mesure obtenu par le calcul de la moyenne des résultats de mesure des champs magnétiques générés par la pluralité d'éléments de génération de champ magnétique pour chaque temps d'un nombre prédéterminé de temps unitaires de mesure les plus récents.

4. Système d'endoscope selon l'une quelconque des revendications 1 à 3,
dans lequel les champs magnétiques générés par la pluralité d'éléments de génération de champ magnétique présentent la même fréquence.

5. Procédé de fonctionnement d'un système d'endoscope qui génère des champs magnétiques en commutant et entraînant de manière sélective une pluralité d'éléments de génération de champ magnétique, et détecte un état d'insertion incluant au moins l'une d'une position ou d'une forme d'une partie d'insertion d'un endoscope sur la base de résultats de mesure des champs magnétiques par des éléments de détection de champ magnétique (25) disposés dans la partie d'insertion (17) de l'endoscope (10), le procédé comprenant :
une étape de commande de génération de champ magnétique pour réaliser une commande de variation d'ordre d'entraînement des éléments de génération de champ magnétique pendant un temps unitaire de mesure prédéterminé entre une pluralité des temps unitaires de mesure.
